# EUROPEAN PATENT APPLICATION

(11) **EP 4 591 872 A1**
(43) Date of publication of application: **30.07.2025**
(21) Application number: 23868597.8
(22) Date of filing: 20.09.2023
(51) Int. Cl.: A61K 35/747, A61K 31/132, A61K 31/194, A61P 1/00, A61P 43/00, A61P 35/00, A23L 33/135, A23K 10/16

(54) **COMPOSITION COMPRISING LACTOBACILLUS PLANTARUM STRAIN FOR IMPROVING INTESTINAL METABOLITE COMPOSITION**

(30) Priority: 20.09.2022 KR 20220118945; 19.06.2023 KR 20230078227
(71) Applicant: Gi Biome Inc., Seongnam-si, Gyeonggi-do 13201 (KR)
(72) Inventor: JANG, Myung Ho, Seoul 05849 (KR); YANG, Bo Gie, Seoul 05849 (KR); KIM, A Ram, Namyangju-si, Gyeonggi-do 12095 (KR)
(74) Representative: Vossius & Partner Patentanwälte Rechtsanwälte mbB
(86) International application number: PCT/KR2023/014313
(87) International publication number: WO 2024/063545

(57) **Abstract**

The present invention relates to a composition comprising a *Lactobacillus plantarum* strain for improving compositions of intestinal metabolites. The *L*. *plantarum* strain according to one aspect can reduce intestinal polyamines (e.g., spermidine), and thus can be effectively used as a pharmaceutical composition, a food (a health functional food), a feed composition, or an anticancer adjuvant, all of which are for preventing, alleviating, and treating cancer, improving the prognosis of cancer, or aiding anticancer treatment.

## Description

### Technical Field

The present invention relates to a composition including a *Lactobacillus plantarum* strain for improving compositions of intestinal metabolites.

### Background Art

The microbiome refers to the collection of microorganisms and genetic information thereof present in a given environment. The microbiome information is being utilized in various fields, such as animals, agriculture, oceans, and environments, in addition to the human body. In particular, advances in human microbiome research based on the development of genetic information analysis and data analysis technologies are expected to drive growth in the diagnostic and healthcare industries.

Human intestinal microorganisms not only break down a variety of substances that human enzymes cannot break down and convert them into nutrients that human cells can absorb, but also are responsible for preventing pathogenic infections by inhibiting the growth of harmful bacteria derived from the outside. Such intestinal bacteria themselves or various metabolites they secrete stimulate numerous immune cells living in the intestinal cells to activate or regulate immune response of the human body. In addition, the human microbiome is also considered the second genome of the human body, with more than 100 times the number and diversity of genes in the commensal microbiota than in humans, making it an increasingly important part of the human genome.

In particular, advances in human microbiome research have led to an increasing number of studies linking the intestinal microorganisms directly or indirectly to a number of human diseases, and a growing number of studies linking the intestinal microorganisms to a number of human cancers.

Meanwhile, polyamines are known to play a role in proliferation, differentiation, and development of eukaryotic organisms. Polyamines are low-molecular-weight organic polycations having two or more amino groups, and examples thereof may include spermine, spermidine, and putrescine which is a diamine precursor of polyamines. Intracellular concentrations of polyamines may be maintained within a certain physiological range through several regulatory mechanisms in normal cells. In addition, polyamine metabolisms are known to be dysregulated in many neoplastic conditions, including cancer. It has been already known in the art that polyamine levels are elevated in various types of cancer and there is a correspondence between polyamine metabolisms and oncogenic pathways, such as the mTOR and RAS pathways. Therefore, polyamines may have potential as therapeutic targets in the prevention and treatment of cancer.

Therefore, there is a need to develop substances to prevent or ameliorate diseases by improving compositions of intestinal metabolites by using the human microbiome.

### Disclosure

### Technical Problem

One aspect is to provide a composition for improving compositions of intestinal metabolites of a subject, the composition including, as an active ingredient, a strain of *Lactobacillus plantarum,* a culture of the strain, a lysate of the strain, or a mixture of the foregoing.

Another aspect is to provide a health functional food for improving compositions of intestinal metabolites of a subject, the health functional food including, as an active ingredient, a strain of *L. plantarum,* a culture of the strain, a lysate of the strain, or a mixture of the foregoing.

Another aspect is to provide a probiotics composition for improving compositions of intestinal metabolites of a subject, the probiotics composition including a strain of *L. plantarum,* a culture of the strain, a lysate of the strain, or a mixture of the foregoing.

Another aspect is to provide a food composition for improving compositions of intestinal metabolites of a subject, the food composition including a strain of *L. plantarum,* a culture of the strain, a lysate of the strain, or a mixture of the foregoing.

Another aspect is to provide a feed composition for improving compositions of intestinal metabolites of a subject, the feed composition including, as an active ingredient, a strain of *L. plantarum,* a culture of the strain, a lysate of the strain, or a mixture of the foregoing.

Another aspect is to provide a pharmaceutical composition for preventing or treating a proliferative disease, especially cancer, the pharmaceutical composition including, as an active ingredient, a strain of *L. plantarum,* a culture of the strain, a lysate of the strain, or a mixture of the foregoing.

Another aspect is to provide an anticancer adjuvant including, as an active ingredient, a strain of *L. plantarum,* a culture of the strain, a lysate of the strain, or a mixture of the foregoing.

Another aspect is to provide a method of improving compositions of intestinal metabolites of a subject, the method including administering an effective amount of a strain of *L. plantarum,* a culture of the strain, a lysate of the strain, or a mixture of the foregoing, to a subject.

Another aspect is to provide a method of preventing or treating cancer, the method including administering an effective amount of a strain of *L. plantarum,* a culture of the strain, a lysate of the strain, or a mixture of the foregoing, to a subject.

Another aspect is to provide use of an effective amount of a strain of *L*. *plantarum,* a culture of the strain, a lysate of the strain, or a mixture of the foregoing, in preparing a formulation for improving compositions of intestinal metabolites of a subject.

Another aspect is to provide use an effective amount of a strain of *L*. *plantarum,* a culture of the strain, a lysate of the strain, or a mixture of the foregoing, in preparing a pharmaceutical formulation or health functional food for preventing or treating cancer.

### Technical Solution

One aspect provides a composition (e.g., a pharmaceutical composition) for improving compositions of intestinal metabolites of a subject, the composition including, as an active ingredient, a strain of *Lactobacillus plantarum,* a culture of the strain, a lysate of the strain, or a mixture of the foregoing.

Another aspect provides a method of improving compositions of intestinal metabolites of a subject, the method including administering an effective amount of a strain of *L. plantarum,* a culture of the strain, a lysate of the strain, or a mixture of the foregoing, to a subject.

Another aspect provides use of an effective amount of a strain of *L. plantarum,* a culture of said strain, a lysate of said strain or a mixture of the foregoing, in preparing a formulation for improving compositions of intestinal metabolites of a subject.

*Lactobacillus* is a genus of aerobic or facultative anaerobic gram-positive rod-shaped bacteria that are widely distributed in nature. Microorganisms belonging to the genus *Lactobacillus* include *Lactobacillus plantarum, Lactobacillus sakei,* and the like. In the research to develop a new strain with excellent anticancer effects, the inventors of the present invention selected *Lactobacillus plantarum* GB104 as a candidate anticancer strain. This strain was deposited at the Korean Collection for Type Cultures of the Korean Research Instituted of Bioscience and Biotechnology on January 14, 2020 under accession number KCTC14107BP. This strain is a probiotic strain, harmless to the human body, and may be used without side effects.

The *Lactobacillus* has been renamed to *Limosilactobacillus* or *Lactiplantibacillus,* and the changed strain names may be used interchangeably in the present specification. For example, *Lactobacillus plantarum* has been renamed to *Lactiplantibacillus plantarum.*

In the present specification, the term "*Lactobacillus plantarum* GB104" may be described in combination with *L. plantarum* GB104 strain or *Lactobacillus plantarum* GB104 strain (accession number: KCTC14107BP).

In an embodiment, the strain may be deposited under accession number KCTC14107BP.

In an embodiment, the strain may include 16S rRNA gene consisting of a nucleotide sequence of SEQ ID NO: 1.

In an embodiment, the strain may include: 16S rRNA consisting of a nucleotide sequence of SEQ ID NO: 1; or 16S rRNA including a nucleotide sequence having nucleotide sequence identity of 97 % or more with the nucleotide sequence of SEQ ID NO: 1. Specifically, the nucleotide sequence identity may be at least 93 %, 95 %, 96 %, 97 %, 98 %, 99 %, 99.5 %, 99.8 %, 99.9 % or 100 % with the nucleotide sequence consisting of SEQ ID NO: 1 of the present specification.

In an embodiment, the strain may be a viable cell, a dead cell, or a cytoplasmic fraction obtained by disrupting the strain, and may be preferably a viable cell.

In the present specification, the term "culture product" may be used interchangeably with "culture supernatant", "supernatant liquid of culture product", "conditioned culture solution", or "culture medium". For example, the culture product may refer to a whole medium containing a strain of the genus *Lactobacillus,* a metabolite thereof, extra nutrients, and the like that are obtained by culturing the strain for a certain period of time in a medium capable of supplying nutrients so that the strain can grow and survive *in vitro.* The culture product may refer to a product obtained by culturing a probiotic strain in a known medium, and the]is product may or may not include the strain itself. The medium may be selected from known liquid media or known solid media. For example, although not limited thereto, the medium may be an MRS liquid medium, a GAM liquid medium, an MRS agar medium, a GAM agar medium, a BL agar medium, or the like.

In the present specification, the term "lysate" may be used interchangeably with "dissolved product", and may refer to a solution of aqueous medium or suspension of cells of a microorganism, such as broken *L. plantarum.* A cell lysate may contain macromolecules, such as DNA, RNA, proteins, peptides, carbohydrates, lipids, etc., and/or small molecules, such as amino acids, sugars, fatty acids, etc., or fractions of the macromolecules or small molecules. In addition, the dissolved product may contain cell debris which may be smooth or granular in structure.

The culture solution may contain a culture solution itself obtained by culturing the strain, a concentrate of the culture solution, or a lyophilized product of the culture solution, or a culture supernatant obtained by removing the strain from the culture solution, a concentrate of the culture supernatant, or a lyophilized product of the culture supernatant.

The culture solution may be obtained by culturing *L. plantarum* in an appropriate medium (e.g., an MRS plate medium) at a temperature of higher than 10 °C or lower than 40 °C, for a certain period of time, for example, 4 to 50 hours.

In an embodiment, the strain, the culture of the strain, or the lysate of the strain may include acetylated spermidine.

In an embodiment, the strain, the culture of the strain, or the lysate of the strain may include polyamine acetyltransferase. More specifically, an enzyme involved in acetylation of polyamines including spermidine may be included.

In an embodiment, acetylated spermidine may be N¹-acetylspermidine, N⁸-acetylspermidine, or N¹,N⁸-diacetylspermidine.

In an embodiment, the strain, the culture of the strain, or the lysate of the strain may include glutaric acid or glutaconic acid.

In an embodiment, the glutaric acid or glutarate may be 2-oxoglutarate or 2-hydroxyglutarate. In addition, in an embodiment, the glutaconic acid may be trans-glutarconic acid.

In one or more embodiments, the improving compositions of intestinal metabolites may include the following:
- a decrease in spermidine, a metabolite in intestines or feces;
- an increase in acetylated spermidine, a metabolite in intestines or feces;
- a decrease in polyamine, a metabolite in intestines or feces;
- a decrease in activity of polyamine synthetase in intestines or feces; and
- an increase in activity of polyamine decomposition enzyme in intestines or feces.

In an embodiment, the polyamine synthetase may be ornithine decarboxylase (ODC), an enzyme that synthesizes spermidine from ornithine in a cell, and the polyamine decomposition enzyme may be spermidine/spermine N¹-acetyltransferase (SSAT), an enzyme that acetylates spermidine, but these are only examples, and the embodiment is not limited thereto. Accordingly, by improving compositions of intestinal metabolites of a subject to which the GB104 strain of the present invention is administered, in the intestines or feces of the subject, the activity of polyamine synthetase (e.g., ODC) may decrease, whereas the activity of polyamine decomposition enzyme (e.g., SSAT) may increase. In the intestines or feces, the levels of ornithine and polyamines (e.g., spermidine) may decrease, whereas the level of acetylated polyamines (e.g., acetylated spermidine) may increase.

The compositions of the intestinal microorganisms, also called gut microbiota, may represent a complex ecosystem in the gastro-intestinal tract. Specifically, the compositions may consist of the entire microbiome in the intestines, including bacteria, yeast, fungi, archaea, and viruses. The intestinal microbiome may maintain multiple functions, including colonic fermentation of dietary fibers, extraction of nutrients, synthesis of certain vitamins, prevention of pathogen-induced colonization, maturation of the intestinal epithelium and immune system, release of metabolites to systemic tissues, and regulation of gastro-intestinal-hormone secretion and neural functions. It is generally recognized that disturbances in the normal balance in the compositions of the intestinal microorganisms may cause a damage in the intestinal barrier integrity, which is often observed in many different diseases. The *L. plantarum* GB104 strain of the present invention may restore and/or maintain compositions of health-beneficial intestinal microorganisms through regulation of the intestinal metabolite profile (specifically, metabolism of polyamines) in a subject. Accordingly, the *L. plantarum* strain described in the present specification may also further provide a composition for restoring and/or maintaining compositions of health-beneficial intestinal microorganisms (or for preventing or treating disorders associated with damaged intestinal integrity).

The metabolism of polyamines may include biosynthesis, catabolism, and transport of polyamines. Natural polyamines may be synthesized in the cytoplasm of all cells. Such biosynthesis begins with L-methionine and L-ornithine, which are amino acids in the urea cycle, and ornithine decarboxylation by ODC results in the formation of putrescine. Putrescine is a polyamine precursor in mammalian cells, producing spermine and spermidine when an aminopropyl group is added by decarboxylated S-adenosylmethionine (dcSAM). The dcSAM is known to be produced by S-adenosylmethionine decarboxylase 1 (SAMDC) or adenosylmethionine decarboxylase 1 (AMD1).

The role of polyamine transport among the metabolisms led to the concept that polyamines are transported into cells via specific polyamine transport systems (PTS). Polyamine uptake via PTS may be upregulated in proliferating cells including tumor cells, suggesting that PTS plays a role in regulating intracellular polyamine concentrations. The levels of polyamine are upregulated in various cancer cell types, and thus polyamines may be general therapeutic targets for cancer treatment. Rapidly growing cells, including tumor cells, may exhibit higher activity of several enzymes involved in the polyamine biosynthesis. The levels of polyamine may be correlated with cancer development, as the levels are elevated in cancer patients. High levels of polyamine are known to be linked to the progression of neuroblastoma, hepatocellular carcinoma (HCC), prostate cancer, lung cancer, breast cancer, stomach cancer, and colorectal cancer (CRC). Decreased level of polyamine are known to induce apoptosis in postmitotic and senescent cells. In addition, polyamines may play a role in establishing tumor immunity which promotes the growth of cancer cells through the excretion of sperms and metabolites thereof. Polyamines are also known to induce acquired chemoresistance to 5-fluorouracil and paclitaxel in CRC and breast cancer. Reduction of polyamines may prevent tumor-induced immunosuppression by enhancing spontaneous IL-2 production, NK cell activity, and restoration of T-lymphocyte populations without affecting tumor polyamines.

In an embodiment, the strain, the culture of the strain, or the lysate of the strain may promote the activity of intestinal immune cells or the expression of a tight junction protein among intestinal cells.

In an embodiment, the activity of the intestinal immune cells may include an increase in the number of activated CD8+ T cells within the immune cells, an increase in the proportion of activated CD8+ T cells within the immune cells, or an increase in the INF-γ secretion, and more specifically, may include an increase in the secretion of cytokines or interferons by the activated immune cells.

In one or more embodiments, the intestinal immune cells may include immune cells within the small intestine or large intestine (e.g., intraepithelial lymphocytes (IEL), small intestinal lamina propria (siLP), colonic lamina propria (cLP)).

In an embodiment, the tight junction protein may include at least one selected from the group consisting of claudin-1, claudin-2, claudin-3, claudin-4, claudin-5, zonula occludens (ZO)-1, ZO-2, ZO-3, and occludin.

Accordingly, the strain, the culture of the strain, or the lysate of the strain may induce the activity of the intestinal immune cells, specifically, the anti-tumor activity by increasing the ratio of cytotoxic T cells (CD8+ T cells) that are directly involved in suppressing the growth of cancer cells among the immune cells in the small intestine or large intestine (e.g., intraepithelial lymphocytes (IEL), small intestinal lamina propria (siLP), colonic lamina propria (cLP)). **In** addition, the gut barrier function may be strengthened by promoting or upregulating the expression of intestinal cell tight junctions.

Therefore, the strain of L. plantarum, the culture of the strain, the lysate of the strain, or a mixture of the foregoing may be provided as a pharmaceutical composition, a food (health functional food), a feed composition, or an anticancer supplement, for preventing, ameliorating, or treating cancer or improving prognosis of cancer by reducing intestinal polyamines.

In an embodiment, the improving compositions of intestinal metabolites of the subject may be for the prevention or treatment of cancer.

The cancer may be any one selected from the group consisting of gastric cancer, liver cancer, lung cancer, colorectal cancer, breast cancer, prostate cancer, ovarian cancer, pancreatic cancer, gallbladder cancer, bile duct cancer, cervical cancer, thyroid cancer, laryngeal cancer, acute myeloid leukemia, brain tumor, neuroblastoma, retinoblastoma, salivary gland cancer, melanoma, bladder cancer, esophagus cancer, head and neck cancer, skin cancer, small intestine cancer, anal cancer, colon cancer, rectal cancer, kidney cancer, blood cancer, and lymphoma. In addition, the colorectal cancer may be malignant tumor occurring in any one site selected from the group consisting of ascending colon, transverse colon, descending colon, sigmoid, and rectal mucosa.

In the present specification, the term "cancer" refers to a physiological condition in animals that is typically characterized by abnormal or uncontrolled cell growth. Cancer and cancer pathology may be associated with, for example, metastasis, interference with normally functioning surrounding cells, release of cytokines or other secretory products at abnormal levels, suppression or enhancement of inflammatory or immunological responses, neoplasia, premalignancy, malignancy, invasion of nearby or distant tissues or organs, such as lymph nodes, etc.

The cancer may be gastrointestinal cancer or non-gastrointestinal cancer.

The gastrointestinal cancer is a malignant tumor occurring in the gastrointestinal tract, such as the esophagus, stomach, small intestine, or large intestine. The gastrointestinal cancer may be, for example, one or more cancers selected from the group consisting of esophageal cancer, gallbladder cancer, liver cancer, bile duct cancer, pancreatic cancer, stomach cancer, small intestine cancer, colorectal cancer, colon cancer, anal cancer and rectal cancer, but is not limited thereto. In an embodiment, the gastrointestinal cancer may be colorectal cancer.

The non-gastrointestinal cancer may include, without limitation, a malignant tumor occurring in organs other than the gastrointestinal tract or digestive system, and for example, may be blood cancer, leukemia, acute myeloid leukemia, neuroblastoma, retinoblastoma, lung cancer, head and neck cancer, salivary gland cancer, melanoma, laryngeal cancer, prostate cancer, breast cancer, bladder cancer, kidney cancer, multiple myeloma, cervical cancer, thyroid cancer, ovarian cancer, urethral cancer, skin cancer, osteosarcoma, glioblastoma, brain tumor, or lymphoma, but is not limited thereto.

In an embodiment of the present invention, the cancer may be colorectal cancer, and the colorectal cancer may include a malignant tumor occurring in one or more sites selected from the group consisting of ascending colon, transverse colon, descending colon, sigmoid colon, and rectal mucosa. The colorectal cancer may be one or more types selected from the group consisting of adenocarcinoma, lymphoma, malignant carcinoid tumor, leiomyosarcoma, Kaposi sarcoma, and squamous cell carcinoma, but is not limited thereto.

The composition according to an embodiment may include 0.001 wt% to 80 wt% of the *L. plantarum* strain based on the total weight of the composition. In addition, a dosage of the *L. plantarum* strain may be 0.01 mg to 10,000 mg, 0.1 mg to 1,000 mg, 1 mg to 100 mg, 0.01 mg to 1,000 mg, 0.01 mg to 100 mg, 0.01 mg to 10 mg, or 0.01 mg to 1 mg. The composition includes the strain in a therapeutically effective amount or in a nutritionally effective concentration. For example, the strain may be present in a dosage of 10³ to 10¹⁶ CFU/g, 10³ to 10¹⁵ CFU/g, 10³ to 10¹⁴ CFU/g, 10³ to 10¹³ CFU/g, 10³ to 10¹² CFU/g, 10⁴ to 10¹⁶ CFU/g, 10⁴ to 10¹⁵ CFU/g, 10⁴ to 10¹⁴ CFU/g, 10⁴ to 10¹³ CFU/g, 10⁴ to 10¹² CFU/g, 10⁵ to 10¹⁶ CFU/g, 10⁵ to 10¹⁵ CFU/g, 10⁵ to 10¹⁴ CFU/g, 10⁵ to 10¹³ CFU/g, 10⁵ to 10¹² CFU/g, 10⁶ to 10¹³ CFU/g, 10⁶ to 10¹² CFU/g, 10⁷ to 10¹³ CFU/g, 10⁷ to 10¹² CFU/g, 10⁸ to 10¹³ CFU/g or 10⁸ to 10¹² CFU/g , or may be included in a culture of an equivalent number of live cells or dead cells. Specifically, for adult patients, 1X10³ to 1X10¹⁶ CFU/g of live bacteria or dead bacteria may be administered in a single dose or in multiple doses. However, the dosage may be prescribed in various ways depending on factors. such as a formulation method, an administration method, the age, weight, gender, pathological condition, diet, administration time, administration route, excretion rate, and response sensitivity of a patient, and a person skilled in the art may appropriately adjust the dosage by considering these factors. The number of administration may be one, or within the limits of clinically acceptable side effects, may be twice or more, and the administration site may be done at one or two or more sites. For animals other than humans, the same dosage per kilogram (kg, body weight) as for humans may be administered, or for example, an amount equivalent to the dosage may be administered based on the volume ratio (e.g., average value) of organs (e.g., the heart) between a target animal and the human. Possible routes of administration may include oral, sublingual, parenteral (e.g., subcutaneous, intramuscular, intraarterial, intraperitoneal, intrathecal, or intravenous), rectal, or topical (including transdermal), inhalation administration, injection, or insertion of an implantable device or material. Examples of the target animal for treatment according to an embodiment may include humans and other mammals for the purpose, and specifically may include humans, monkeys, mice, rats, rabbits, sheep, cows, dogs, horses, pigs, etc. In an embodiment, the composition may include a killed, dried strain, and may be administered at a dosage of 1 g to 10 g, 0.5 g to 1.5 g, 2.5 g to 3.5 g, or 4.5 g to 5.5 g at a time, and may be administered once a day to three times a day.

In the present specification, the term "therapeutically effective amount" may refer to an amount of an anticancer agent for the methods and uses of the present invention or a pharmaceutical composition including an anticancer agent for the methods and uses of the present invention that will produce biological or medical responses or desired therapeutic effects in a patient as sought by researchers, physicians, or other clinicians. The therapeutically effective amount of an anticancer agent may vary depending on factors such as a disease state, age, gender, and body weight of a subject, and the ability of the anticancer agent to produce a desired response in a subject. The therapeutically effective amount may be an amount at which therapeutically beneficial effects surpass any toxic or harmful effects.

The pharmaceutical composition according to an embodiment may include a pharmaceutically acceptable carrier and/or additive. For example, the pharmaceutical composition may include sterile water, physiological saline, general buffer (e.g., phosphoric acid, citric acid, other organic acids, etc.), a stabilizer, a salt, an antioxidant (e.g., ascorbic acid, etc.), a surfactant, a suspending agent, an isotonic agent, or a preservative. For topical administration, an organic material including a biopolymer, an inorganic material including hydroxyapatite, specifically a collagen matrix, a polylactic acid polymer or copolymer, a polyethylene glycol polymer or copolymer, and a chemical derivative thereof may be combined.

In an embodiment, the pharmaceutical composition may be prepared in oral formulations.

In an embodiment, the oral formulations may include tablets, pills, capsules, lozenges, granules, powders, suspensions, sachets, or syrups.

When the pharmaceutical composition according to an embodiment is formulated in a dosage form suitable for injection, the strains of the genus *Lactobacillus* may be dissolved or dispersed in a pharmaceutically acceptable carrier, or may be frozen in a solution in which the strains are dissolved or dispersed.

The pharmaceutical composition according to an embodiment may include a suspending agent, a solubilizing agent, a stabilizer, an isotonic agent, a preservative, an adsorption inhibitor, a surfactant, a diluent, an excipient, a pH adjuster, an analgesic agent, a buffer, a reducing agent, an antioxidant, and the like may be appropriately included when necessary according to the administration method or formulation of the pharmaceutical composition. Pharmaceutically acceptable carriers and agents suitable for the present invention, including those exemplified above, are described in detail in the document [Remington's Pharmaceutical Sciences, 19th ed., 1995]. The pharmaceutical composition may be formulated into a unit dosage form or prepared in a multi-dose container by formulating a pharmaceutically acceptable carrier and/or excipient according to the method easily carried out by a person having ordinary skill in the art to which the present invention pertains. Here, the formulation may be in the form of a solution, suspension, or emulsion in an oily or aqueous medium, or in the form of a powder, a granule, a tablet, or a capsule.

The pharmaceutical composition may be administered in a pharmaceutically effective amount. In the present specification, the term "pharmaceutically effective amount" refers to an amount sufficient to treat a disease with a reasonable benefit/risk ratio applicable to medical treatment, and an effective dose level may be determined based on disease types and severity of a patient, drug activity, drug sensitivity, an administration time, an administration route, and an excretion rate, duration of treatment, factors including concomitant drugs, and other factors well known in the medical field. The composition of the present invention may be administered as an individual therapeutic agent or in combination with other therapeutic agents, may be administered sequentially or simultaneously with a conventional therapeutic agent, and may be administered in a single dose or multiple doses. In consideration of all of the factors described above, it is important to administer an amount that will produce the maximum effect in the least amount without side effects, which can be readily determined by those skilled in the art..

Another aspect is to provide a health functional food for improving compositions of intestinal metabolites of a subject, the health functional food including a strain of *L. plantarum,* a culture of the strain, a lysate of the strain, or a mixture of the foregoing, as an active ingredient.

In an embodiment, the improving compositions of intestinal metabolites of the subject may include improving intestinal health by increasing beneficial intestinal bacteria, suppressing harmful bacteria, regulating immunity, or improving bowel movement.

In an embodiment, the strain, the culture of the strain, or the lysate of the strain may include acetylated spermidine or polyamine acetyltransferase.

In an embodiment, acetylated spermidine may be N¹-acetylspermidine, N⁸-acetylspermidine, or N¹,N⁸-diacetylspermidine.

In an embodiment, the strain, the culture of the strain, or the lysate of the strain may include glutaric acid or glutaconic acid.

In an embodiment, the glutaric acid or glutarate may be 2-oxoglutarate or 2-hydroxyglutarate. In addition, in an embodiment, the glutaconic acid may be trans-glutarconic acid.

In an embodiment, the improving compositions of intestinal metabolites may include the following:
- a decrease in spermidine, a metabolite in intestines or feces;
- an increase in acetylated spermidine, a metabolite in intestines or feces;
- a decrease in polyamine, a metabolite in intestines or feces;
- a decrease in activity of polyamine synthetase in intestines or feces; and
- an increase in activity of polyamine decomposition enzyme in intestines or feces.

In an embodiment, the strain, the culture of the strain, or the lysate of the strain may promote or upregulate the activity of intestinal immune cells or the expression of a tight junction protein among intestinal cells.

In an embodiment, the activity of the intestinal immune cells may include an increase in the number of activated CD8+ T cells within the immune cells, an increase in the proportion of activated CD8+ T cells within the immune cells, or an increase in the INF-γ secretion, and more specifically, may include an increase in the secretion of cytokines or interferons by the activated immune cells.

In one or more embodiments, the intestinal immune cells may include immune cells within the small intestine or large intestine (e.g., IEL, siLP, cLP).

In an embodiment, the tight junction protein may include at least one selected from the group consisting of claudin-1, claudin-2, claudin-3, claudin-4, claudin-5, ZO-1, ZO-2, ZO-3, and occludin.

In an embodiment, the health functional food may be an oral preparation.

The strain, the culture of the strain, the lysate of the strain or the mixture of the foregoing, the administration route, the administration dosage, and the administration dose are as described above.

In an embodiment, the health functional food may additionally contain a food acceptable carrier.

In the present disclosure, the term "food acceptable" refers to exhibiting characteristics that are not toxic to cells or humans exposed to the composition.

In the present specification, the term "improvement" may refer to any act that at least reduces a parameter associated with the condition being treated, for example, the severity of symptoms. Here, the health functional food may be used simultaneously with or independently of a therapeutic agent for the prevention or amelioration of cancer, either before or after the onset of the disease.

In the health functional food, an active ingredient may be added to the food as is or used in combination with other foods or food ingredients, and may be used appropriately according to conventional methods. An amount of the active ingredient being mixed may be appropriately determined according to the purpose of use (for prevention or amelioration purposes). Generally, in the preparation of food or beverage, the health functional food may be added in an amount of about 15 wt% or less, more specifically about 10 wt% or less, relative to the raw material. However, in the case of long-term intake for health and hygiene purposes or for health control purposes, the amount may be below the ranges above.

The health functional food may be formulated in one selected from the group consisting of tablets, pills, granules, powders, capsules, and liquid formulations, by additionally containing one or more of carriers, diluents, excipients, and additives. Food products to which the composition according to an embodiment may be added may include various foods, powders, granules, tablets, capsules, syrups, beverages, gums, teas, vitamin complexes, and health functional food products.

Specific examples of the carriers, excipients, diluents, and additives may include at least one selected from the group consisting of lactose, dextrose, sucrose, sorbitol, mannitol, erythritol, starch, acacia gum, calcium phosphate, alginate, gelatin, calcium phosphate, calcium silicate, microcrystalline cellulose, polyvinylpyrrolidone, cellulose, polyvinylpyrrolidone, methylcellulose, water, sugar syrup, methylcellulose, methylhydroxy benzoate, propylhydroxy benzoate, talc, magnesium stearate, and mineral oil.

In addition to containing the above-described active ingredient, the health functional food may contain other ingredients as essential ingredients without special limitation. For example, the health functional food may contain additional ingredients, such as various flavorings or natural carbohydrates, as in regular beverages. Examples of the natural carbohydrates may include common sugars including: monosaccharides such as glucose, fructose, etc.; disaccharides such as maltose, sucrose, etc.; and polysaccharides such as dextrin, cyclodextrin, etc., and sugar alcohols including xylitol, sorbitol, erythritol, etc. In addition to those described above, natural flavorings (thaumatin, stevia extracts (e.g., rebaudioside A, glycyrrhizin, etc.)), and synthetic flavorings (e.g., saccharin, aspartame, etc.) may be advantageously used as flavorings. Proportions of the natural carbohydrates may be appropriately determined by a person skilled in the art.

In addition to those described above, the health functional food according to an aspect may contain various types of nutritional supplements, vitamins, minerals (e.g., electrolytes), flavors including synthetic flavors and natural aromas, colorants, extenders (e.g., cheese, chocolate, etc.), pectic acid and salts thereof, alginic acid and salts thereof, organic acids, protective colloidal thickeners, pH regulators, stabilizers, antiseptics, glycerin, alcohols, carbonating agents used in carbonate beverages, etc. These components may be used independently or in combination, and the proportion of these additives may also be appropriately selected by those skilled in the art.

The health functional food may be provided in combination with a conventionally known health functional food for preventing or ameliorating cancer or with another conventional health functional food, and the health functional food for preventing or ameliorating cancer may be a conventionally known health functional food for preventing or ameliorating metabolic diseases, an existing health functional food, or a newly developed health functional food.

When the health functional food contains other health functional foods having a preventive or ameliorative effect on cancer, it is important that the amounts are combined to achieve maximum effect in the least amount without side effects, which can be readily determined by a person skilled in the art.

Food compositions for preventing or ameliorating cancer may be prepared in a variety of forms, including all forms of functional foods, nutritional supplements, health foods, and food additives, and food compositions of these types may be prepared according to conventional methods known in the art.

The compositions of the present invention may be regarded as food supplements. Food supplements, also known as dietary supplements or nutritional supplements, may be regarded as another specific pharmaceutical product. Such food supplements are intended to supplement a diet and provide nutrients or beneficial ingredients that may not be consumed from a normal diet or may not be consumed in a sufficient amount. Most food supplements are regarded as a food, but sometimes they are considered drugs, natural health products, or nutraceutical products. Within the meaning of the present invention, the food supplements may include health functional foods. The food supplements are usually sold over the counter without prescription. When the food supplements are in the form of pills or capsules, the same excipients used in medicines may be contained. However, the food supplements may be also in the form of foods fortified with some nutrients (e.g., infant formula). Thus, in one or more embodiments, the composition of the present invention may be a food supplement.

The composition according to the present invention may be administered as is or mixed with a suitable edible liquid or solid, or may be freeze-dried in the form of tablets, pills, capsules, lozenges, granules, powders, suspensions, sachets, syrups or in the form of unit doses. It may also be in the form of monodoses of a lyophilized composition that are mixed in a separate liquid container provided prior to administration.

The composition of the present invention may be contained in various edible products and foods, including dairy products, for infants. In the present document, the term "edible product" may refer to, in a broad sense, any product that can be ingested in any form by an animal (e.g., a product that can perceived by the sense organs). The term "food product" may be understood as an edible product that provides nutritional support to the body. In particular, food products of interest are food supplements and infant formulas. The food may preferably include carrier materials such as oatmeal gruel, lactic acid fermented foods, resistant starch, dietary fibers, carbohydrates, proteins, and glycosylated proteins. In some embodiments, bacterial cells of the present invention may be homogenized with other ingredients, such as cereals or powdered milk, to form infant formulas.

Another aspect is to provide a feed composition for improving compositions of intestinal metabolites of a subject, the feed composition including a strain of *L. plantarum,* a culture of the strain, a lysate of the strain, or a mixture of the foregoing, as an active ingredient.

The strain, the culture of the strain, the lysate of the strain or the mixture of the foregoing, the administration route, the administration dosage, and the administration dose are as described above.

The feed composition may be prepared by adding the mixed strain composition in an appropriate effective concentration range according to various feed preparation methods known in the art, and may be used as a feed additive composition for the purpose of preventing or ameliorating age-related diseases.

The "feed" may refer to any natural or artificial diet, meal, etc., or ingredients of the meal, that is intended for or suitable for eating, ingesting, or digesting by an animal. Types of the feed are not particularly limited, and any feed commonly used in the relevant technical field may be used. Non-limiting examples of the feed may include: plant feeds such as grains, root fruits, food processing by-products, algae, fibers, pharmaceutical by-products, fat and oils, starches, gourds, or grain by-products; and animal feeds such as proteins, fat-free substances, minerals, fat and oils, single-cell proteins, zooplanktons, or food stuffs.

Another aspect provides an anticancer adjuvant including, as an active ingredient, a strain of *L. plantarum,* a culture of the strain, a lysate of the strain, or a mixture of the foregoing.

The strain, the culture of the strain, the lysate of the strain, or the mixture of the foregoing, the administration route, the administration dosage, and the administration dose are as described above.

In this specification, the term "adjuvant" refers to a substance that assists the efficacy of a main drug, i.e., an anticancer drug, to improve and/or enhance the therapeutic effect, or prevents or alleviates the harmful effects of the main drug. The strain of *L. plantarum* of the present invention may improve anticancer effects of other anticancer agents without presenting a burden to the body on its own, by improving the compositions of intestinal metabolites.

The other anticancer agents may be selected from the group consisting of chemotherapeutic agents, targeted anticancer agents, antibody therapeutic agents, immunotherapy agents, and combinations thereof, for chemotherapy as a concomitant conventional treatment.

In the present specification, the term "chemotherapeutic agent" is also referred to as an antineoplastic agent or a cytotoxic agent. The chemotherapeutic agent is a general term for drugs that exhibit anticancer activity by acting directly on DNA to block DNA replication, transcription, and translation processes, or by interfering with synthesis of nucleic acid precursors in metabolic pathways and inhibiting cell division. Specifically, the chemotherapeutic agent may be any one selected from the group consisting of an alkylating agent, a microtubule inhibitor, an antimetabolite, and a topoisomerase inhibitor. Such antitumor drugs exhibit cytotoxicity by acting on normal cells as well as tumor cells. The chemotherapeutic agent may be used for maintenance therapy. In addition, in the present specification, the term "maintenance therapy" refers to a treatment of cancer with drugs after initial chemotherapy to prevent or delay the recurrence of cancer.

In the present specification, the term "targeted anticancer agent" refers to a therapeutic agent that specifically kills cancer cells by blocking signals involved in the growth and development of cancer by targeting specific proteins or specific genetic changes that are abundant only in cancer cells. The targeted anticancer agent may be classified into monoclonal antibodies that react outside a cell and small molecule substances that act inside a cell. The monoclonal antibodies are anticancer agents that block cancer cell-inducing signals transmitted to outside a cell, acting on initiation signals related to proliferation, death, etc., and the small molecule substance may act on complex signaling occurring within a cell.

Specifically, examples of proteins being targeted may include epidermal growth factor receptor (EGFR), vascular endothelial growth factor receptor (VEGFR), CD20, CD38, RNAK-L, BTK, Bcr-abl, PDGFR/FGFR family, MEK/RAF, HER2/Neu, ubiquitin, JAK, MAP2K, ALK, PARP, tumor growth factor β receptor (TGFβR), proteasome, Bcl-2, C-Met, VR1, VR2, VR3, c-kit, AXL, RET, Braf, DNA methyltransferase (DNMT), CDK4/6, STING, etc.

In the present specification, the term "antibody therapeutic agent" refers to a therapeutic agent that exhibits an anticancer effect by utilizing an antibody that recognizes a cancer cell-specific protein as an antigen. Examples of the antibody therapeutic agent may include Cetuximab, Trastuzumab, Emtansine, Rituximab, Ibritumomab, Tositumomab, Brentuximab, Ofatumumab, Obinutuzumab, Necitumumab, Bevacizumab, Ramucirumab, Nivolumab, Pembrolizumab, Atezolizumab, Durvalumab, Ipilimumab, etc.

In the present specification, the term "immunotherapy agent" refers to a substance that interferes with the activity of immune checkpoint proteins that inhibit the differentiation, proliferation, and activity of immune cells, and the immunotherapy agent is known to eliminate cancer cells by preventing cancer cells from exerting the ability of evading the immune system. The immunotherapy agent may be an antibody against any one selected from the group consisting of 2B4, 4-1BB (CD137), AaR, B7-H3, B7-H4, BAFFR, BTLA, CD2, CD7, CD27, CD28, CD30, CD40, CD80, CD83 ligand, CD86, CD160, CD200, CDS, CEACAM, CTLA-4, GITR, HVEM, ICAM-1, KIR, LAG-3, LAIR1, LFA-1 (CD 11 a/CD 18), LIGHT, NKG2C, NKp80, OX40, PD-1, PD-L1, PD-L2, SLAMF7, TGFRp, TIGIT, Tim3, and VISTA. More specifically, the immunotherapy agent may be any one selected from the group consisting of anti-CTLA-4 antibody, anti-PD-1 antibody, anti-PD-L1 antibody, anti-PD-L2 antibody, anti-B7-H4 antibody, anti-HVEM antibody, anti-TIM3 antibody, anti-GAL9 antibody, anti-LAG3 antibody, anti-VISTA antibody, anti-KIR antibody, anti-BTLA antibody, and anti-TIGIT antibody, but is not limited thereto.

The strain according to an embodiment, the culture of the strain, or the lysate of the strain may be administered in combination with the other anticancer agents.

In the present specification, the term "combination therapy" or "combined administration" or the expression "in combination" refers to any form of simultaneous or concurrent treatments using at least two separate therapeutic agents. Components of the combination therapy may be administered simultaneously, sequentially, or in any order. The components may be administered in different dosages, at different frequency of administration, or via different routes, as appropriate.

Specifically, the combined administration may refer to simultaneous administration of: the strain of *L. plantarum* and an anticancer agent or antibiotic, or administration of the strain of *L. plantarum* followed by administration of an anticancer agent or antibiotic. The combination therapy according to the present invention may be defined as providing synergistic effects if the efficacy, as measured by, for example, degree of response, rate of response, time to disease progression, or duration of survival, is therapeutically superior to the efficacy that would be obtained by administering one or others of the components of the combination therapy at conventional doses. For example, the efficacy of combination therapy is considered synergistic if it is therapeutically superior to the efficacy of each of the components used alone. In particular, a synergistic effect is considered to exist if it is possible to reduce the usual dose of the strain of *L. plantarum* and the anticancer agent or antibiotic, without harming one or more of the degree of response, the rate of response, the time to disease progression and the survival data, especially without harming the duration of response, and with fewer and/or less problematic side effects than those that would occur if each component was used at its usual dose.

In the present specification, the meaning of the expression "administered simultaneously" is not particularly limited, and refers that the components of the combination therapy are administered substantially simultaneously, for example, as a mixture or in immediately subsequent sequences.

In the present specification, the meaning of the expression "administered sequentially" is not particularly limited, and refers that the components of the combination therapy are not administered simultaneously, but are administered one after another or in groups with a specific time interval between administrations. The time interval may be the same or different between administrations of each of the components of the combination therapy, and may be selected from a range of, for example, 2 minutes to 96 hours, 1 day to 7 days, or 1 week, 2 weeks or 3 weeks. Generally, the time interval between administrations may range from a few minutes to several hours, such as 2 minutes to 72 hours, 30 minutes to 24 hours, or 1 to 12 hours. For example, the time interval may range from 24 to 96 hours, 12 to 36 hours, 8 to 24 hours, and 6 to 12 hours.

### Advantageous Effects

According to the strain of *Lactobacillus plantarum* of one aspect, the strain can reduce intestinal polyamines (e.g., spermidine), and thus may be useful in a pharmaceutical composition, food (health functional food), feed composition, or anticancer adjuvant for preventing, ameliorating, or treating cancer or ameliorating prognosis of cancer.

### Description of Drawings

FIG. 1 is a graph showing a difference in relative contents of metabolites in GB104 culture supernatant and MRS culture supernatant (control group), plotted by a metabolic pathway using CE-TOF-MS.
FIG. 2 is a table showing changes in contents of trans-glutaconic among metabolites in GB104 culture supernatant, a strain of *L. plantarum* F0077, and MRS culture supernatant (control group), the changes being expressed as fold changes relative to the overall average.
FIG. 3 is a table showing changes in contents of spermidine and three types of acetylated spermidines among metabolites in GB104 culture supernatant and MRS culture supernatant (control group), the changes being expressed as fold changes relative to the overall average.
FIG. 4 is a graph showing a difference in relative contents of spermidine and three types of acetylated spermidines among metabolites in GB104 culture supernatant and MRS culture supernatant (control group).
FIG. 5 is a graph showing a difference in relative contents of spermidine and acetylated spermidine, after treatment of the colorectal cancer cell line HCT116 with GB104 culture supernatant and MRS culture supernatant (control group).
FIG. 6 is a graph showing changes in expression of ODC which is a polyamine synthetase and SSAT which is a polyamine decomposition enzyme, after treatment of colorectal cancer cell lines HCT116 and HT-29 with GB104 culture supernatant and MRS culture supernatant (control group), wherein ODC represents ornithine decarboxylase, and SSAT represents spermidine/spermine N¹-acetyltransferase.
FIG. 7 is a graph showing changes in relative spermidine contents in feces, after treatment of each of GB104 and PBS (control group) in mice implanted with colorectal cancer cells MC38.
FIG. 8 is a graph showing changes in the contents of N¹-acetylspermidine/spermidine and ornithine in tumors, after treatment of each of GB104 and PBS (control group) with mice implanted with colorectal cancer cells MC38.
FIG. 9 is a graph showing changes in the spermidine contents in feces, after treatment of each of GB104 and PBS (control group) with mice treated with antibiotics.
FIG. 10 is a graph showing changes in the number of immune cells in intestines, after treatment of each of GB104 and PBS (control group) with mice treated with antibiotics.
FIG. 11 is a graph showing changes in expression of tight junction-related genes in intestinal tissues, after treatment of each of GB104 and PBS (control group) with mice treated with antibiotics, wherein ZO represents zonula occludens.
FIG. 12 is a graph showing viability of cells, after treatment of each of GB104 and a culture supernatant of other strains with colorectal cancer cell line HCT116.
FIG. 13 is a graph showing a cell cycle, after treatment of each of GB104 and a culture supernatant of a comparison strain (WCFS1) with colorectal cancer cell line HCT116.
FIG. 14 is a graph showing apoptosis, after treatment of each of GB104 and a culture supernatant of a comparison strain (WCFS1) with colorectal cancer cell line HCT116.

### Mode for Invention

Hereinafter, preferable Examples are presented to help understanding of the present disclosure. However, the following examples are only presented for easier understanding of the present disclosure, and the contents of the present disclosure are not limited by the following examples. Examples can undergo various modifications, and thus examples are not limited to those disclosed below and can be implemented in various forms.

### Example 1. Isolation and identification of Lactobacillus plantarum GB104 strain

Isolation and identification of *L. plantarum* GB104 strain was performed according to the methods described in KR 10-2020-0186738 and KR 10-2022-0080567. These documents are incorporated herein by reference in their entirety.

Briefly, the *L. plantarum* GB104 strain was isolated from vaginal samples of healthy women visiting a clinic for a health checkup. First, vaginal samples were collected with a swab and inoculated by streaking onto a Rogosa SL (MRS) plate medium, and then cultured in an anaerobic chamber at 37 °C for 48 hours. When the bacterial colonies grew, single colonies were subcultured onto a fresh MRS plate medium for pure isolation. After the pure isolation, the strains were cultured by using an MRS medium. Next, among the cultured strains, a *L. plantarum* GB104 strain was finally selected for its inhibitory effect on adipocyte accumulation and low cytotoxicity. To identify the finally selected *L. plantarum* GB104 strain, the 16S rRNA gene sequence obtained by PCR using primers targeting the 16S rRNA gene was analyzed by Sanger sequencing, and the 16S rRNA sequence of the *L. plantarum* GB104 strain was represented by SEQ ID NO: 1. The inventors of the present invention named the GB104 bacteria as "L. *plantarum* GB104" (accession number: KCTC 14107BP), which was deposited in the Korean Collection for Type Cultures (KCTC) of the Korea Research Institute of Bioscience and Biotechnology on January 14, 2020. In addition, the *L. plantarum* has been renamed to *Lactiplantibacillus plantarum.* In the following examples, the renamed strain names of the existing strains are described interchangeably.

### Experimental Example 1. Results of polyamine and trans-glutaconic acid analysis and content comparison in GB104 culture supernatant

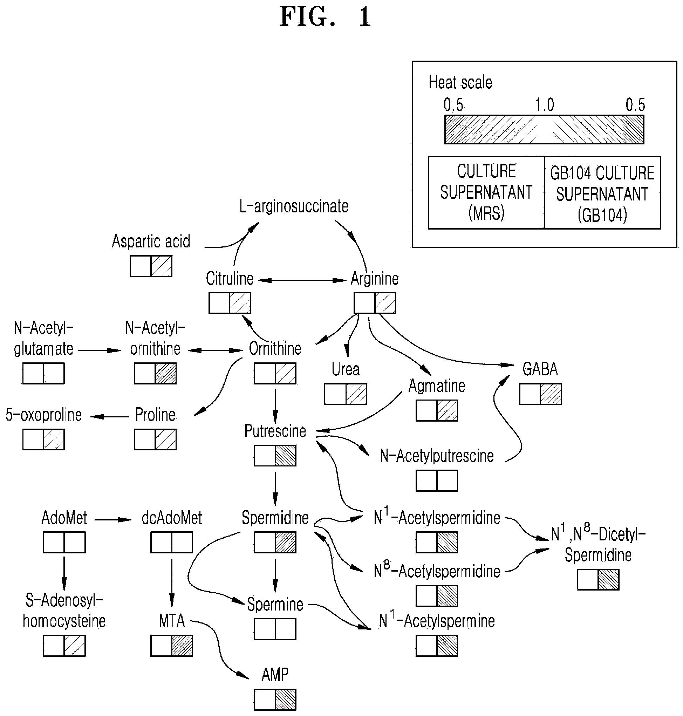

Metabolome analysis of MRS culture supernatant (control group) and GB104 culture supernatant was performed by Human Metabolome Technologies (HMT). Milli-Q water containing internal standards was added to MRS culture supernatant and GB104 culture supernatant samples, and CE-TOF-MS analysis was performed on the samples. Metabolome analysis of cations and anions was performed by using the Agilent CE-TOF-MS system (Agilent Technologies Inc.),and the results are shown in FIG. 1. Separation of metabolites was achieved through a fused silica capillary. Putative metabolites analyzed by HMT were identified through the HMT internal library.

FIG. 1 is a graph showing the difference in relative contents of metabolites in GB104 culture supernatant and MRS culture supernatant (control group), plotted by a metabolic pathway using CE-TOF-MS.

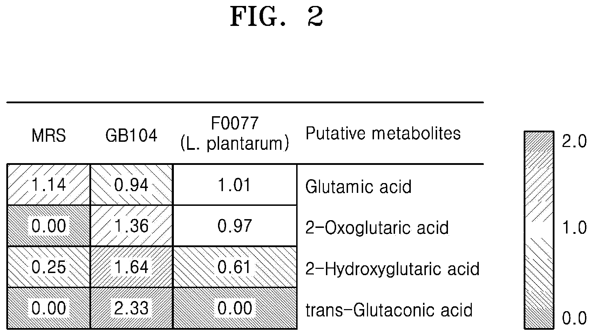

FIG. 2 is a table showing changes in contents of trans-glutaconic among metabolites in GB104 culture supernatant, *L. plantarum* F0077 strain, and MRS culture supernatant (control group), the changes being expressed as fold changes relative to the entire bacteria.

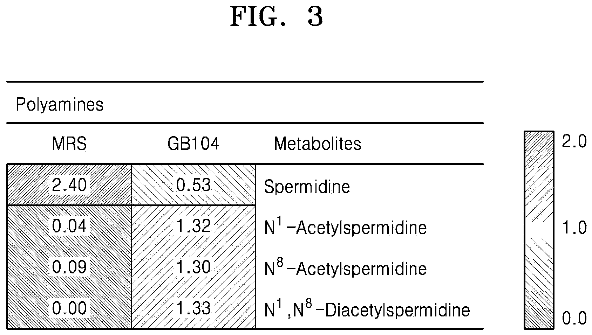

FIG. 3 is a table showing changes in the contents of spermidine and three types of acetylated spermidines among metabolites in GB104 culture supernatant and MRS culture supernatant (control group), the changes being expressed as fold changes relative to the overall average.

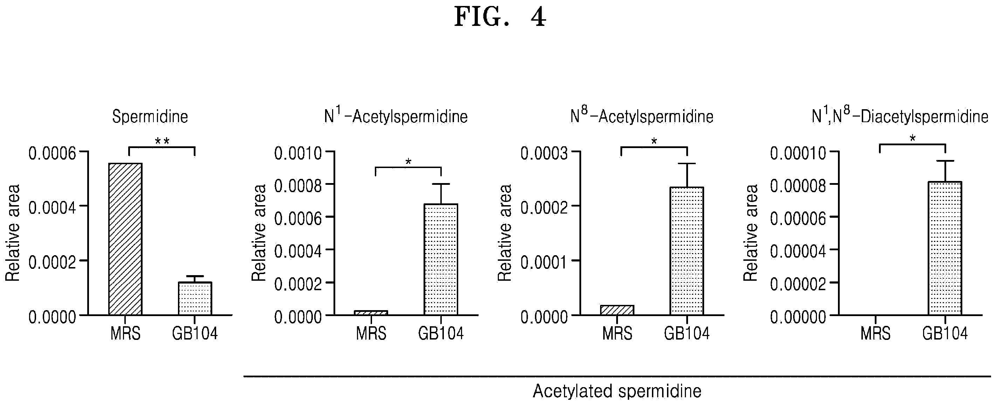

FIG. 4 is a graph showing the difference in relative contents of spermidine and three types of acetylated spermidines among metabolites in GB104 culture supernatant and MRS culture supernatant (control group).

As shown in FIGS. 1 to 4, a total of 296 metabolites were analyzed, including both identified substances and unknown substances through the HMT internal library. Metabolites that were knocked down by the GB104 were further analyzed by using the OPLS-DA model, which is used to determine the differences between two groups, and 87 metabolites were selected out of a total of 296 metabolites. These metabolites included a variety of metabolites, including amino acids, peptides, nucleosides, nucleotides, organic acids, sugars, etc. Notably, changes in polyamines that are known to be involved in the proliferation of cancer cells were remarkable. The GB104 acetylated spermidine which is a polyamine component, resulting in a decrease in spermidine, but an increase in acetylated spermidine.

### Experimental Example 2. Changes in metabolites and gene expression in cancer cells following treatment with GB104 culture supernatant

Effects of *L. plantarum* GB104 culture supernatant on the changes in metabolites and gene expression in human colorectal cancer cell lines were determined.

Human colorectal cancer cell line (HCT116) was seeded into each well of a 6-well plate at a concentration of 2X10⁵ cells, and cultured for 24 hours. Then, a culture supernatant of the *L. plantarum* GB104 strain was treated at a concentration of 10 % and cultured for 24 hours under conditions of 37 °C and 5 % CO². Regarding the culture supernatant, *L. plantarum* strains were cultured for 8, 16, and 24 hours, respectively, and precipitated by centrifugation, and only the supernatant was collected and then filtered through a 0.22 µm filter to obtain the supernatant for each culture time.

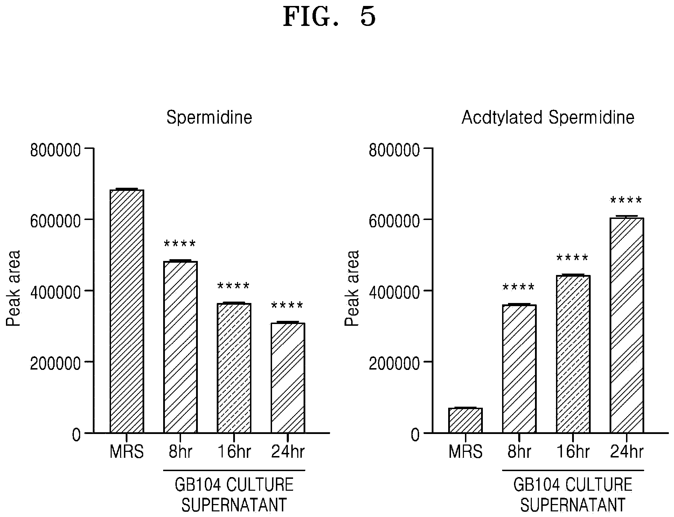

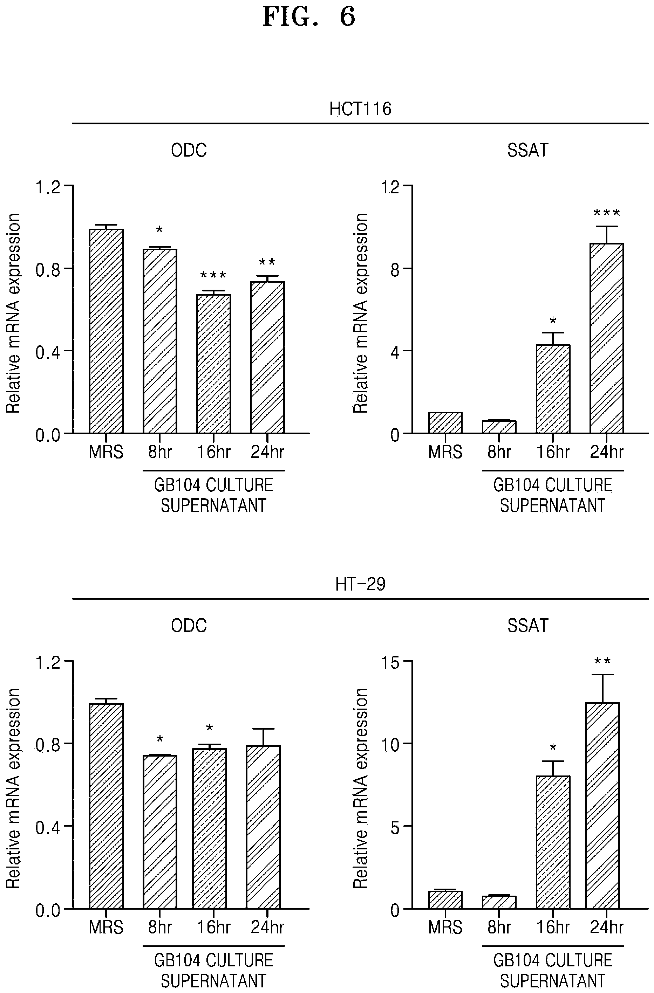

The changes in the content of polyamines in cancer cells were determined by extracting metabolites and performing HPLC-DAD analysis on HCT116 cell pellets treated with the GB104 supernatant for each culture time, and the results are each shown in peak area values in FIG. 5. In addition, the changes in the expression of genes that inhibit synthesis and promote degradation of polyamines by the GB104 metabolites were confirmed based on the mRNA expression levels by using a QuantStudio 3 real-time PCR instrument, after RNA extraction and synthesis of cDNA in the HCT116 and HT-29 cell pellets treated with the supernatant for each culture time, and the results are shown in FIG. 6. The polyamines, which are involved in the growth and proliferation of cells, are synthesized in a particularly high content in cancer cells, and play an important role in the proliferation of cancer cells. The polyamines are synthesized in cells from ornithine by ornithine decarboxylase (ODC) and degraded by acetylation by spermidine/spermine N1-acetyltransferase (SSAT). It has been reported that increased SSAT expression in cancer cells inhibits the proliferation of cancer cells, and that high SSAT expression in cancer tissues leads to good prognosis for anticancer therapy.

FIG. 5 is a graph showing the difference in relative contents of spermidine and acetylated spermidine, after treatment of the colorectal cancer cell line HCT116 with GB104 culture supernatant and MRS culture supernatant (control group).

FIG. 6 is a graph showing changes in expression of ODC which is a polyamine synthetase and SSAT which is a polyamine decomposition enzyme, after treatment of colorectal cancer cell lines HCT116 and HT-29 with GB104 culture supernatant and MRS culture supernatant (control group), wherein ODC represents ornithine decarboxylase, and SSAT represents spermidine/spermine N 1-acetyltransferase.

As shown in FIG. 5, in the HCT116 colon cancer cells treated with the GB104 culture supernatant of GB104, spermidine decreased and acetylspermidine increased, in a culture time-dependent manner. Accordingly, it was confirmed that the GB104 acetylates polyamines.

As shown in FIG. 6, in both colorectal cancer cell lines HCT116 and HT-29 treated with the GB104 culture supernatant, the expression of ODC, a polyamine synthetase, decreased and the expression of SSAT, which is a polyamine decomposition enzyme, increased. Accordingly, it was confirmed that the GB104 inhibits cancer cell proliferation induced by polyamines by inhibiting the synthesis of polyamines and increasing the decomposition of polyamines in cancer cells.

### Experimental Example 3. Spermidine content changes in feces of mouse model

### 3.1. Comparison of spermidine content changes in feces of MC-38 colorectal cancer mouse model

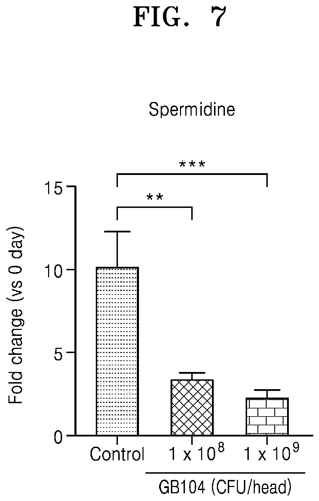

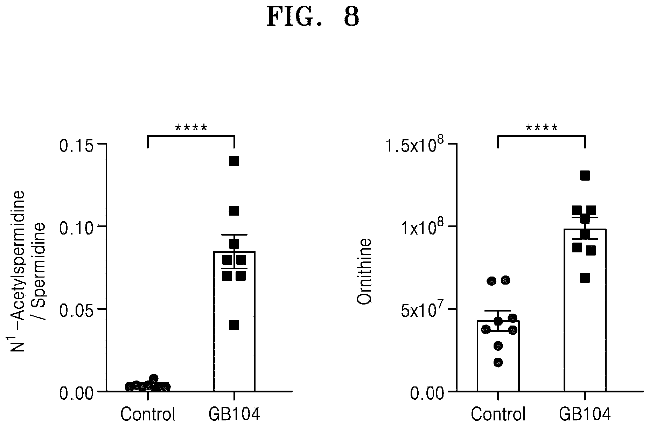

In the MC-38 allograft model of colorectal carcinoma, the changes in the spermidine content in feces and tumors induced by administration of the *L*. *plantarum* GB104 strain were confirmed. A tumor model was established by subcutaneously injecting the colorectal cancer cell line MC-38 into the right flank of 7-week-old c57BL/6 mice at 100 µL of 2 x 10⁵ cells per mouse. On 5 day after the injection of tumor cells, only mice having tumor sizes within a range of 20 to 40 mm³ were selected and randomly assigned to each group, and then the *L*. *plantarum* GB104 strain was administered orally to the animal models at 1x10⁹ CFU per mouse daily from 6 day until shortly before the end of the experiment. Feces were collected from mice in all groups on 6 day of the injection of tumor cells (just before oral administration of the GB104) (samples of 0 day), and feces were collected again from mice in all groups on 12 day (samples of 7 day). At the end of the experiment, on 20 day of the injection of tumor cells, tumor samples were obtained from mice. Polyamines were extracted and derivatized from the mouse feces and tumor samples, and then subjected to HPLC-DAD analysis. As a result, polyamines were detected in the feces and tumor samples, and it was confirmed that changes in the polyamine content were caused by the GB104. The changes in the spermidine content in feces were expressed as fold by dividing the area of the spermidine peak in the feces samples after 7 days of oral administration of the GB104 by the area of the area of the spermidine peak in the feces samples collected immediately before oral administration of the GB104. These values were compared among groups, and the results are shown in FIG. 7. The changes in the acetylspermidine content in tumors were expressed by dividing the area of the N¹-acetylspermidine peak by the area of the spermidine peak, and the changes in the ornithine content were expressed by the area value of the ornithine peak as shown in FIG. 8.

FIG. 7 is a graph showing the changes in relative spermidine contents in feces, after treatment of each of the GB104 and PBS (control group) in mice implanted with the colorectal cancer cells MC38.

FIG. 8 is a graph showing the changes in the contents of N¹-acetylspermidine/spermidine and ornithine in tumors, after treatment of each of the GB104 and PBS (control group) with mice implanted with the colorectal cancer cells MC38.

As shown in FIG. 7, the GB104 reduced the spermidine content in a dose-dependent manner, and in particular, the changes in the spermidine content were significantly reduced in the GB104 (treatment dose 1 x 10⁹ CFU/200 µL/head) administration group compared to the control group.

As shown in FIG. 8, the percentage of acetylspermidine was high in tumors of the mice treated with the GB104, indicating that the GB104 not only acetylated polyamines to weaken the proliferation ability of cancer cells, but also facilitated the decomposition of polyamines. In addition, ornithine, a substrate of the polyamine synthetase ODC, was increased in tumors of the mice treated with the GB104, indicating that the GB104 inhibited the activity of polyamine synthetase.

### 3.2. Comparison of spermidine content changes in feces of antibiotic-treated colorectal cancer mouse model

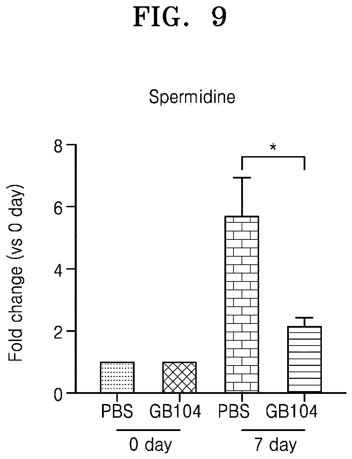

In the antibiotic-treated mouse model, the changes in the spermidine content in feces and tumors induced by administration of the *L. plantarum* GB104 strain were confirmed. Sterilized drinking water was mixed with antibiotics and kept in a dark place, and the mice were allowed to freely consume this drinking water for 7 days. Then, the antibiotic-treated drinking water was refreshed every 2 to 3 days. From 7 day onward, the mice were provided with sterilized plain drinking water instead of the antibiotics-containing drinking water until the end of the experiment. Freeze-dried GB104 was suspended in D-PBS to obtain a dose of 1 x 10⁹ CFU/head, which was then administered orally at 200 µL per mouse daily for 7 days from the end of the antibiotic treatment using an oral zonde. Feces were collected from mice in all groups at the end of the antibiotic treatment and immediately before administration of the GB104 (samples of 0 day), and feces were collected again from mice in all groups on 7 day of oral administration of the GB104 (samples of 7 day). Polyamines were extracted and derivatized from mouse fecal samples, and HPLC-DAD analysis was performed. Spermidine was detected in feces samples, and it was confirmed that the changes in the spermidine content were caused by the GB104. The changes in the spermidine content were expressed as fold by dividing the area of the spermidine peak in the feces samples after 7 days of oral administration of the GB104 by the area of the area of the spermidine peak in the feces samples collected immediately before oral administration of the GB104. These values were compared among groups, and the results are shown in FIG. 9.

FIG. 9 is a graph showing the changes in the spermidine content in feces, after treatment of each of the GB104 and PBS (control group) with mice treated with antibiotics.

As shown in FIG. 9, the spermidine content in feces was decreased by the antibiotic treatment, but increased on 7 day at which the antibiotics became less effective. However, this increase in spermidine was significantly reduced by the GB104.

### Experimental Example 4. Changes in the number of immune cells in intestines following GB104 administration

In the antibiotic-treated mouse model, the changes in immune cells in the intestines induced by administration of the *L. plantarum* GB104 strain were confirmed.

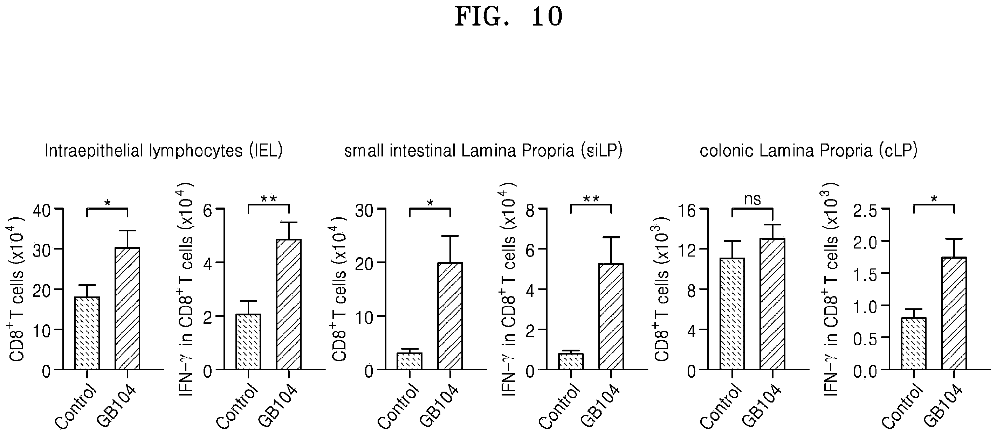

Sterilized drinking water was mixed with antibiotics and kept in a dark place, and the mice were allowed to freely consume this drinking water for 7 days. Then, the antibiotic-treated drinking water was refreshed every 2 to 3 days. From 7 day onward, the mice were provided with sterilized plain drinking water instead of the antibiotics-containing drinking water until the end of the experiment. Freeze-dried GB104 was suspended in D-PBS and administered orally at a dose of 1 x 10⁹ CFU/head/200 µL daily for 14 days from the end of the antibiotic treatment. Afterwards, tissues of the small intestine and the large intestines were obtained from the mice, and the small intestine was cleared of fat and Peyer's patch. The intestinal tissue was opened by a longitudinal incision, washed with PBS, and cut into 1 to 2 cm long pieces. To remove epithelial cells, intraepithelial lymphocytes (IELs) were additionally isolated from the supernatant filtered from the small intestine after stirring at 37 °C for 20 minutes in FACS buffer (PBS containing 3 % FBS, 20 mM HEPES, 100 U/ml penicillin, 100 µg/ml streptomycin, 1 mM sodium pyruvate, and 10 mM EDTA). Pieces of the intestinal tissue with epithelial cells removed were washed with PBS, minced, and stirred in an enzyme medium (RPMI 400 medium containing 3 % FBS with 400 U/ml collagenase D and 10 µg/ml DNAse I of the small intestine and 800 U/ml collagenase D and 10 µg/ml DNAse I of the large intestine, 20 mM HEPES, 100 U/ml penicillin, 100 µg/ml streptomycin, 1 mM sodium pyruvate, and 1 mM NEAA) at 37 °C for 45 minutes. The enzyme reaction was stopped by treating with 10 mM EDTA, and the cells passed through a strainer were resuspended in a 40 % percoll solution and centrifuged with 75 % percoll solution added on top. After the centrifugation, the middle layer was recovered and lamina propria (LP) cells were isolated. The isolated immune cells were stained with fluorescent antibodies specific for markers of the cells to be identified, and then analyzed by using the FACSymphony equipment, and the results are shown in FIG. 10.

FIG. 10 is a graph showing the changes in the number of immune cells in the intestines, after treatment of each of the GB104 and PBS (control group) with mice treated with antibiotics.

As shown in FIG. 10, as a result of confirming the changes in immune cells in the small intestine and the large intestine, it was confirmed that, compared to the control group, the proportion of cytotoxic T cells (CD8+ T cells), a type of immune cells known to directly inhibit cancer growth, was significantly increased in a group administered with the *L. plantarum* GB104 strain, and that IFN-γ secreted from these cells was also increased significantly. Accordingly, it was confirmed that the *L. plantarum* GB104 strain affects the increase and activation of CD8+ antitumor immune T cells.

### Experimental Example 5. Changes in expression of tight junction-related genes in intestinal tissues following GB104 administration

In the antibiotic-treated mouse model, changes in the expression of tight junction-related genes in the intestinal tissues in response to administration of the *L. plantarum* GB104 strain were confirmed.

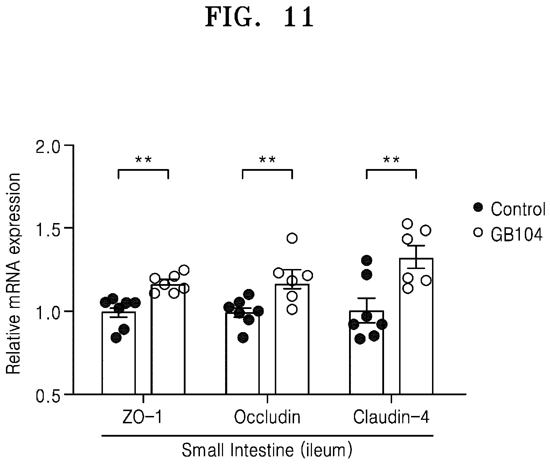

Sterilized drinking water was mixed with antibiotics and kept in a dark place, and the mice were allowed to freely consume this drinking water for 7 days. Then, the antibiotic-treated drinking water was refreshed every 2 to 3 days. From 7 day onward, the mice were provided with sterilized plain drinking water instead of the antibiotics-containing drinking water until the end of the experiment. Freeze-dried GB104 was suspended in D-PBS to obtain a dose of 1 x 10⁹ CFU/head, which was then administered orally at 200 µL per mouse daily for 14 days from the end of the antibiotic treatment using an oral zonde. On 14 day of oral administration of the GB104, the ileum tissue was extracted and shredded to extract RNA which was then synthesized in to cDNA. The mRNA expression levels of tight junction-related genes (ZO-1, occludin, claudin-4) were confirmed by using a QuantStudio 3 real-time PCR Instrument, and the results are shown in FIG. 11.

FIG. 11 is a graph showing the changes in the expression of tight junction-related genes in the intestinal tissues, after treatment of each of the GB104 and PBS (control group) with mice treated with antibiotics, wherein ZO represents zonula occludens.

As shown in FIG. 11, the GB104 increased the expression level of the tight junction-related genes (ZO-1, occludin, and claudin-4) in the small intestine tissues. Accordingly, it was confirmed that the GB104 can strengthen the gut barrier function by increasing the expression of the tight junction-related genes.

### Experimental Example 6. Confirmation of cancer cell viability and cancer cell killing effects according to treatment with GB104 strain culture supernatant

### 6.1. Confirmation of cancer cell viability according to treatment with GB104 strain culture supernatant

By using the human colorectal cancer cell line, various *L. plantarum* culture supernatants containing the *L. plantarum* GB104 strain were each treated, and the cell viability was screened by MTT assay.

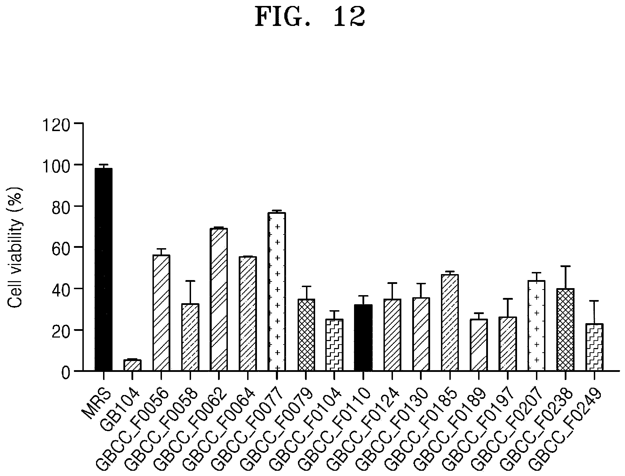

The human colorectal cancer cell line HCT116 cells were seeded into each well of a 96-well plate at a density of 2X10³ cells and cultured for 24 hours. Then, in a culture medium containing DFMO and aminoguanidine, a culture supernatant of various *L. plantarum* strains treated at a concentration of 10 % and cultured for 72 hours under conditions of 37 °C and 5 % CO². To obtain the culture supernatant, the *L. plantarum* strain was cultured in an MRS medium and precipitated by centrifugation, and only the supernatant was collected and then filtered through a 0.22 µm filter. By using cell proliferation kit I (MTT) (Roche), the MTT solution was treated to 0.5 mg/mL and cultured for another 4 hours to dissolve the purple formazan crystals produced in living cells by metabolic activity in a solubilization solution. Then, the absorbance was measured at 570 nm, and the results are shown in FIG. 12.

FIG. 12 is a graph showing the viability of cells, after treatment of each of the GB104 and the culture supernatant of other strains with the colorectal cancer cell line HCT116.

As shown in FIG. 12, the same inhibitory effects on the growth of cancer cells was not observed in the *L. plantarum* culture supernatant. In particular, the GB104 culture supernatant showed a reduction in cancer cell viability of about 95 %, confirming that it was most effective in inhibiting the growth of cancer cells.

### 6.2. Confirmation of cancer cell cycle according to treatment with GB104 strain culture supernatant

By using the human colorectal cancer cell line, the *L. plantarum* GB104 culture supernatant was treated, and the changes in the cancer cell cycle were confirmed by using a flow cytometer.

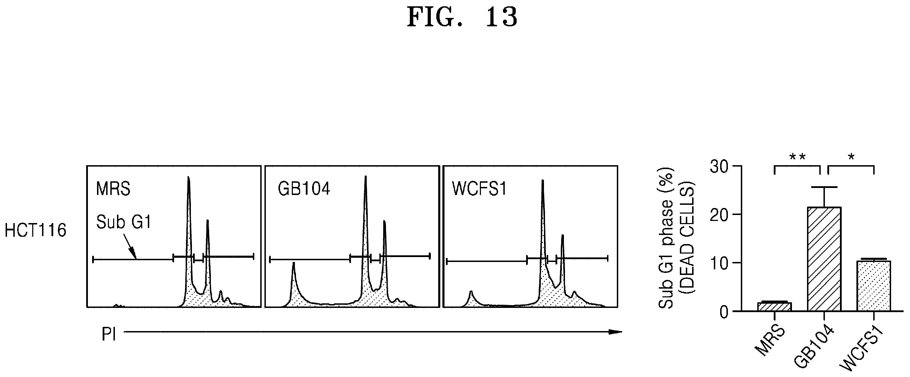

The human colorectal cancer cell line HCT116 cells were seeded into each well of a 6-well plate at a density of 5X10⁴ cells and cultured for 24 hours. Then, in a culture medium containing DFMO and aminoguanidine, a culture supernatant of the *L. plantarum* GB104 strain was treated at a concentration of 10 % and cultured for 48 hours under conditions of 37 °C and 5 % CO². To obtain the culture supernatant, the *L. plantarum* strain was cultured in an MRS medium and precipitated by centrifugation, and only the supernatant was collected and then filtered through a 0.22 µm filter. After 48 hours, cells were detached by treating with trypsin-EDTA, harvested by centrifugation, fixed with 70 % ethanol, and then stored at 4°C for at least 1 hour. After treating with RNase A and staining with propidium iodine (PI), the degree of cancer cell death was confirmed by cell cycle analysis using a flow cytometer (BD FACSymphony A3 Cell Analyzer), and the results are shown in FIG. 13.

FIG. 13 is a graph showing the cell cycle, after treatment of each of the GB104 and a culture supernatant of a comparison strain (WCFS1) with the colorectal cancer cell line HCT116.

As shown in FIG. 13, it was confirmed that the sub-G1 region of cells treated with the *L. plantarum* GB104 culture supernatant increased about 9.8-fold compared to cells of the MRS-treated control group. Accordingly, it was confirmed that the GB104 induces the death of cancer cells.

### 6.3. Confirmation of cancer cell killing effects according to treatment with GB104 strain culture supernatant

By using the human colorectal cancer cell line, the *L. plantarum* GB104 culture supernatant was treated, and the cancer cell killing effects were confirmed by using a flow cytometer.

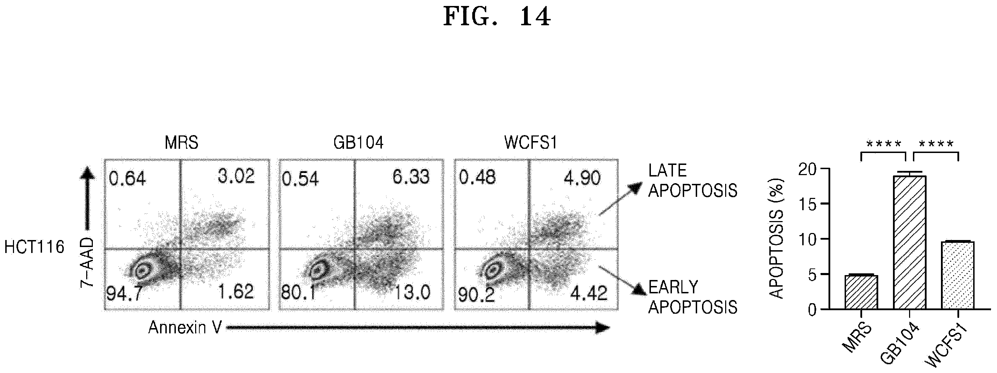

The human colorectal cancer cell line HCT116 cells were seeded into each well of a 6-well plate at a density of 5X10⁴ cells and cultured for 24 hours. Then, in a culture medium containing DFMO and aminoguanidine, a culture supernatant of the *L. plantarum* GB104 strain was treated at a concentration of 10 % and cultured for 48 hours under conditions of 37 °C and 5 % CO². To obtain the culture supernatant, the *L. plantarum* strain was cultured in an MRS medium and precipitated by centrifugation, and only the supernatant was collected and then filtered through a 0.22 µm filter. After 48 hours, the cells were detached by treating with trypsin-EDTA and then harvested by centrifugation. The cells were mixed with 100 µL binding solution by using the FITC annexin V apoptosis detection kit with 7-AAD (BioLegend), and 5 µL FITC annexin V and 5 µL 7-AAD were added thereto for a reaction at room temperature for 15 minutes in a shaded condition. Fluorescence measurements to confirm the death of cancer cells were performed by using a flow cytometer (BD FACSymphony A3 cell analyzer), and the results are shown in FIG. 14. Normally living cells are not labeled with annexin V or 7-AAD, whereas cells in early apoptosis are labeled with annexin V but not with 7-AAD. Cells in late apoptosis can be identified by labeling with both Annexin V and 7-AAD.

FIG. 14 is a graph showing apoptosis, after treatment of each of the GB104 and the culture supernatant of a comparison strain (WCFS1) with the colorectal cancer cell line HCT116.

As shown in FIG. 14, it was confirmed that the early apoptosis and the late apoptosis of cancer cells treated with the *L. plantarum* GB104 culture supernatant increased about 4-fold compared to cells of the MRS-treated control group. Referring to the results above, it was confirmed that the GB104 was involved in the induction of early apoptosis of cancer cells.

The foregoing descriptions are only for illustrating the present disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

### [Accession Number]

Name of deposit institution : Korea Research Institute of Bioscience and Biotechnology
Accession Number: KCTC14107BP
Accession Date: 20200114

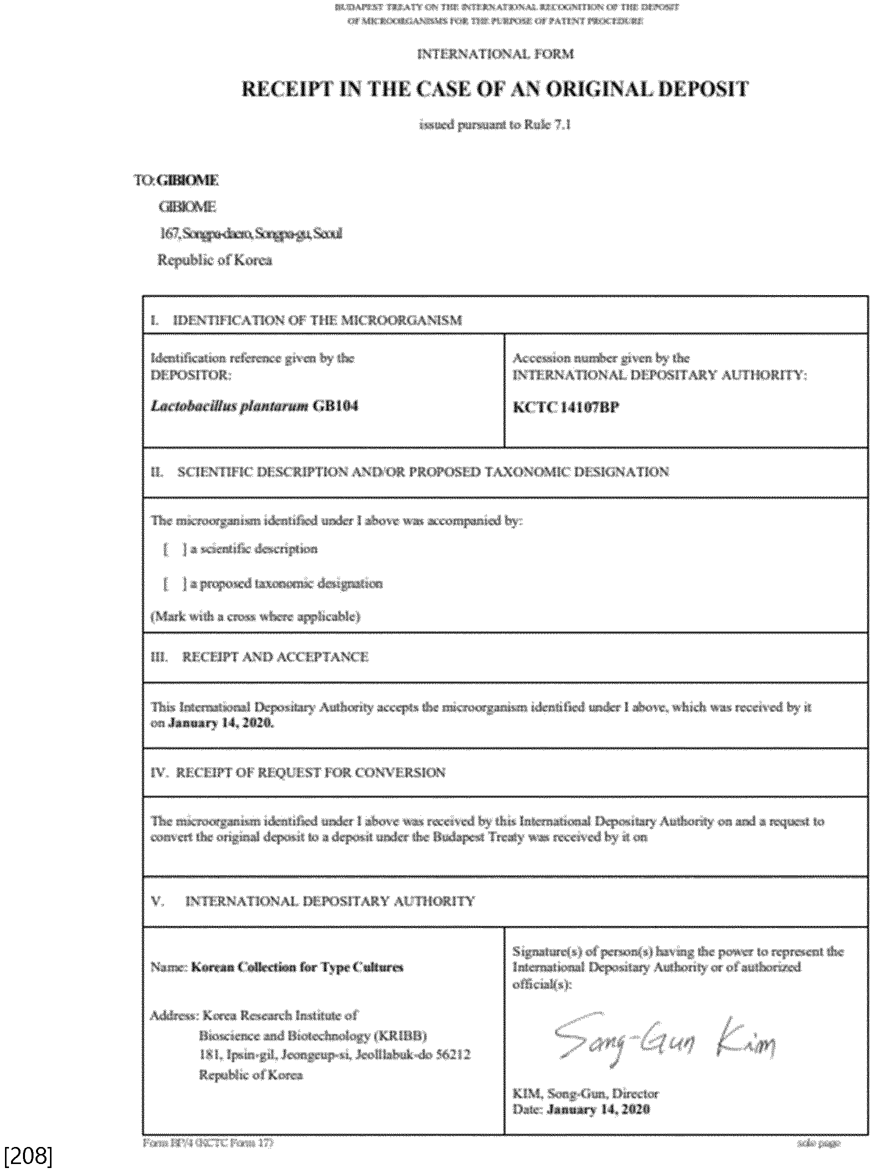
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO:GIBIOME
GIBIOME
167,Songpa-daero,Songpa-gu,Seoul
Republic of Korea

I. IDENTIFICATION OF THE MICROORGANISM
Identification reference given by the
DEPOSITOR:
Lactobacillus plantarum GB104 | Accession number given by the
INTERNATIONAL DEPOSITARY AUTHORITY:
KCTC 14107BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on January 14, 2020.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):
KIM, Song-Gun, Director
Date: January 14, 2020
Form BP/4 (KCTC Form 17) sole page
[208]

## Claims

1. A composition for improving compositions of intestinal metabolites of a subject, the composition comprising, as an active ingredient, a strain of *Lactobacillus plantarum,* a culture of the strain, a lysate of the strain, or a mixture of the foregoing.

2. The composition of claim 1, wherein the strain is deposited under accession number KCTC14107BP.

3. The composition of claim 1, wherein the strain comprises 16S rRNA of SEQ ID NO: 1.

4. The composition of claim 1, wherein the strain, the culture of the strain, or the lysate of the strain comprises acetylated spermidine.

5. The composition of claim 4, wherein the acetylated spermidine is N¹-acetylspermidine, N⁸-acetylspermidine, or N¹,N⁸-diacetylspermidine.

6. The composition of claim 1, wherein the strain, the culture of the strain, or the lysate of the strain comprises polyamine acetyltransferase.

7. The composition of claim 1, wherein the strain, the culture of the strain, or the lysate of the strain comprises glutaric acid or glutaconic acid.

8. The composition of claim 7, wherein the glutaric acid is 2-oxoglutaric acid or 2-hydroxyglutaric acid.

9. The composition of claim 1, wherein the improving compositions of intestinal metabolites comprises at least one of the following characteristics:
- a decrease in spermidine, a metabolite in intestines or feces;
- an increase in acetylated spermidine, a metabolite in intestines or feces;
- a decrease in polyamine, a metabolite in intestines or feces;
- a decrease in activity of polyamine synthetase in intestines or feces; and
- an increase in activity of polyamine decomposition enzyme in intestines or feces.

10. The composition of claim 1, wherein the strain, the culture of the strain, or the lysate of the strain promotes activity of intestinal immune cells or expression of a tight junction protein among intestinal cells.

11. The composition of claim 10, wherein the activity of intestinal immune cells includes: an increase in the number of activated CD8+ T cells in the immune cells; or an increase in INF-γ secretion.

12. The composition of claim 10, wherein the tight junction protein is at least one selected from the group consisting of zonula occludens (ZO)-1, ZO-2, ZO-3, occludin, claudin-1, claudin-2, claudin-3, and claudin-4.

13. A pharmaceutical composition for preventing or treating cancer, the pharmaceutical composition comprising, as an active ingredient, a strain of *Lactobacillus plantarum,* a culture of the strain, a lysate of the strain, or a mixture of the foregoing.

14. The pharmaceutical composition of claim 13, wherein the strain is included in an amount of 10³ to 10¹⁶ cfu/g.

15. The pharmaceutical composition of claim 13, wherein the pharmaceutical composition is an oral formulation.

16. The pharmaceutical composition of claim 13, wherein the cancer is any one selected from the group consisting of gastric cancer, liver cancer, lung cancer, colorectal cancer, breast cancer, prostate cancer, ovarian cancer, pancreatic cancer, gallbladder cancer, bile duct cancer, cervical cancer, thyroid cancer, laryngeal cancer, acute myeloid leukemia, brain tumor, neuroblastoma, retinoblastoma, salivary gland cancer, melanoma, bladder cancer, esophagus cancer, head and neck cancer, skin cancer, small intestine cancer, anal cancer, colon cancer, rectal cancer, kidney cancer, blood cancer, and lymphoma.

17. The pharmaceutical composition of claim 16, wherein the colorectal cancer occurs in any one site selected from the group consisting of ascending colon, transverse colon, descending colon, sigmoid, and rectal mucosa.

18. A health functional food for improving compositions of intestinal metabolites of a subject, the health functional food comprising, as an active ingredient, a strain of *Lactobacillus plantarum,* a culture of the strain, a lysate of the strain, or a mixture of the foregoing.

19. The health functional food of claim 18, wherein the strain, the culture of the strain, or the lysate of the strain comprises acetylated spermidine or polyamine acetyltransferase.

20. The health functional food of claim 18, wherein the strain, the culture of the strain, or the lysate of the strain comprises glutaric acid or glutaconic acid.

21. The health functional food of claim 18, wherein the improving compositions of intestinal metabolites comprises the following:
- a decrease in spermidine, a metabolite in intestines or feces;
- an increase in acetylated spermidine, a metabolite in intestines or feces;
- a decrease in polyamine, a metabolite in intestines or feces;
- a decrease in activity of polyamine synthetase in intestines or feces; and
- an increase in activity of polyamine decomposition enzyme in intestines or feces.

22. The health functional food of claim 18, wherein the strain, the culture of the strain, or the lysate of the strain promotes activity of intestinal immune cells or expression of a tight junction protein among intestinal cells.

23. The health functional food of claim 18, wherein the health functional food is an oral formulation.

24. An anticancer adjuvant comprising, as an active ingredient, a strain of *Lactobacillus plantarum,* a culture of the strain, a lysate of the strain, or a mixture of the foregoing.

25. A feed composition for improving compositions of intestinal metabolites of a subject, the feed composition comprising, as an active ingredient, a strain of *Lactobacillus plantarum,* a culture of the strain, a lysate of the strain, or a mixture of the foregoing.

26. A method of improving compositions of intestinal metabolites of a subject, the method comprising administering an effective amount of a strain of *Lactobacillus plantarum,* a culture of the strain, a lysate of the strain, or a mixture of the foregoing, to a subject in need thereof.

27. A method of preventing or treating cancer, the method comprising administering an effective amount of a strain of *Lactobacillus plantarum,* a culture of the strain, a lysate of the strain, or a mixture of the foregoing, to a subject in need thereof.

28. Use of a composition comprising a strain of *Lactobacillus plantarum,* a culture of the strain, a lysate of the strain, or a mixture of the foregoing, in preparing a formulation for improving compositions of intestinal metabolites of a subject.

29. Use of a composition comprising a strain of *Lactobacillus plantarum,* a culture of the strain, a lysate of the strain, or a mixture of the foregoing, in preparing a pharmaceutical formulation for preventing or treating cancer.

30. Use of a composition comprising a strain of *Lactobacillus plantarum,* a culture of the strain, a lysate of the strain, or a mixture of the foregoing, in preparing a health functional food for preventing or treating cancer.
